(19) Europäisches Patentamt
European Patent Office
Office européen des brevets

(11) Veröffentlichungsnummer: **0 212 623**
**A2**

(12) **EUROPÄISCHE PATENTANMELDUNG**

(21) Anmeldenummer: 86111473.4

(22) Anmeldetag: 19.08.86

(51) Int. Cl.4: **A23L 1/221**, A23L 2/36, A23L 1/24, A23L 1/39, A23C 9/13

(30) Priorität: **30.08.85 DE 3531130**
**13.02.86 DE 3604515**

(43) Veröffentlichungstag der Anmeldung: **04.03.87 Patentblatt 87/10**

(84) Benannte Vertragsstaaten: **AT BE CH DE FR GB IT LI LU NL SE**

(71) Anmelder: **Hopstabil Hopfenverarbeitungs-Gesellschaft mbH**
**Ingolstädter Strasse 24**
**D-8069 Wolnzach(DE)**

(72) Erfinder: **Klüsters, Paul**
**Grabengasse 10**
**D-8068 Pfaffenhofen/Ilm(DE)**
Erfinder: **Paul, Herbert**
**Dr. Baybergerstrasse 17**
**D-8069 Geisenfeld(DE)**

(74) Vertreter: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Aromazusatz zu Lebensmitteln und Getränken.**

(57) Es wird ein Aromazusatz zu Lebensmitteln und Getränken, ausgenommen Bier und Ale, beschrieben, der gleichzeitig bakteriostatisch wirkt und gekennzeichnet ist durch einen Gehalt an Humulon und/oder alpha-Isohumulon sowie deren Derivate, gegebenenfalls in Kombination mit Hopfenölen.

EP 0 212 623 A2

## Aromazusatz zu Lebensmitteln und Getränken

Die Erfindung betrifft einen aromatisierend und bakteriostatisch wirkenden Zusatz zu Lebensmitteln und Getränken, ausgenommen Bier.

Aus US-PS 3,486,906 und GB-PS 1,362,695 ist bekannt, Bier und Ale zweiwertige Metallkomplexe von Isohumulon sowie Hopfenöle als Aroma-bzw. Bitterstoff zuzugeben. Isohumulon hat einen hohen Bitterwert. Beim Hopfenkochen gehen die im Hopfen enthaltenen Humulone in cis-und trans-Isohumulone über; diese Isosäuren sind leichter löslich und bitterer als die Ausgangssubstanzen. Ausserdem ist bekannt, durch eine Isomerisierungsreaktion Erdalkali-Humulate in die entsprechenden Isohumulate zu überführen, und diese dann der Würze oder dem Bier zuzugeben.

Nach einem von der Anmelderin erarbeiteten Verfahren erfolgt die Zugabe von Isohumulon zur Würze oder zum Bier in Form der freien alpha-Isosäure. Hierzu wird auf DE-OS 35 13 183.7 verwiesen.

DE-OS 35 13 169.1 der Anmelderin beschreibt ein Verfahren zur Herstellung von freier alpha-Isohumulonsäure aus einem Hopfenextrakt, wonach letzterer im Verhältnis 1:2 bis 1:10 mit einer wässrigen Lösung unter Rühren bei erhöhter Temperatur vermischt und dabei ein Zwei-Phasen-System erhalten wird, daraufhin die wässrige Phase abgetrennt wird und aus dieser durch Zugabe eines Erdalkalisalzes die Humulone ausgefällt werden; daran schliesst sich eine Isomerisierung der Erdalkalihumulate zu den entsprechenden Erdalkaliisohumulaten sowie die Umwandlung der letzteren zu den freien Isohumulonen an.

Auch die Verwendung von Humulon und dessen Derivaten, die durch Extraktion aus Hopfen gewonnen werden, in Bier zur Erzielung eines bitter-würzigen Aromas ist bekannt.

Unter den Bitterstoffen des Hopfens sind Humulone bzw. alpha-Säuren von grosser Bedeutung. Die im Hopfen zu 2,2 bis 13,5 % (bezogen auf das Trockengewicht) enthaltenen Humulone gehen bei Hopfenkochen, wie vorstehend beschrieben, in cis-und trans-Isohumulone über; diese Isosäuren sind leichter löslich als die Ausgangssubstanzen.

Hopfenöle, die gemäss der Erfindung in Kombination mit Humulonen und/oder Isohumulonen verwendet werden können, sind im Lupulin zu 0,2 bis 2,5 % als etherische Öle enthalten. Auch sie gehen beim Erwärmen, z.B. beim Hopfenkochen, aufgrund ihrer Wasserdampfflüchtigkeit zum grossen Teil verloren. Um dem Bier die erwünschte "Hopfenblume" zu verleihen, werden diesem im Lagertank oder kurz vor Kochende Hopfenölpräparate zugegeben.

Hopfenöle können bei der extraktiven Aufbereitung von Hopfen gewonnen werden, wobei z.B. von der im vorstehend beschriebenen Verfahren der Anmelderin (DE-OS 35 13 169) erhaltenen öligen Phase, die nach dem Extrahieren des Hopfenextraktes mit einer wässrigen Lösung verbleibt, ausgegangen werden kann.

Die Anmelderin hat nun in überraschender Weise gefunden, dass sich Humulone und/oder Isohumulone, gegebenenfalls in Kombination mit Hopfenölen, als Aromazusatz für eine Reihe von Lebensmitteln und Getränken eignen, wobei dieser Zusatz in unterschiedlichem Masse jeweils geschmacksverstärkend und/oder geschmacksverändernd wirkt. Dabei wirken die Humulone und Isohumulone, bzw. Derivate derselben, aromatisierend und bakteriostatisch.

Aufgabe der vorliegenden Erfindung ist es, einen aromatisierend und bakteriostatisch wirkenden Zusatz zu Lebensmitteln und Getränken, ausgenommen Bier und Ale, zur Verfügung zu stellen. Von besonderer Bedeutung ist dabei, dass dieser Zusatz einen erprobten Naturstoff darstellt.

Die vorstehende Aufgabe wird gemäss der Erfindung durch ein aromatisierend und bakteriostatisch wirkenden Zusatz zu Lebensmitteln und Getränken, ausgenommen Bier und Ale, gelöst, der gekennzeichnet ist durch einen Gehalt an Humulon und/oder alpha-Isohumulon bzw. Derivate derselben, gegebenenfalls in Kombination mit Hopfenölen.

Unter dem vorstehend genannten Ausdruck "Humulon und dessen Derivate" sind die im Hopfen als Bitterstoffe enthaltenen Humulone der folgenden Zusammensetzung zu verstehen:

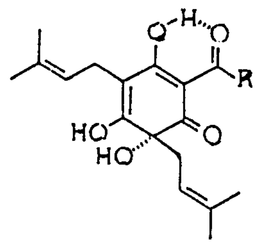

R = $CH_2CH(CH_3)_2$: Humulon

R = $CH(CH_3$: Cohumulon

R = $CH(CH_3)CH_2CH_3$: Adhumulon

R = $CH_2CH_2CH(CH_3)_2$: Prähumulon

R = $CH_2CH_3$: Posthumulon

Beim Erwärmen, z.B. beim Hopfenkochen, erfahren die vorstehend angegebenen Humulone eine Isomerisierung zu den sogenannten Iso-alpha-Säuren, e.g. cis-und trans-Isohumulon, die den nachstehend angegebenen Formeln entsprechen:

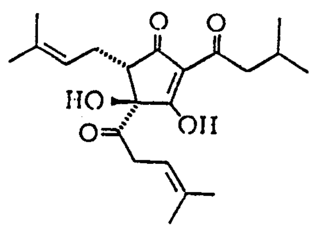

cis-Isohumulon

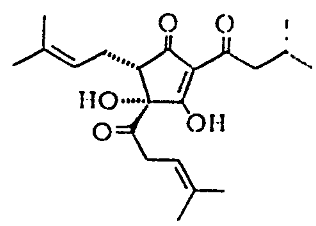

trans-Isohumulon

Weitere Isomerisierungsprodukte bilden die cis-und trans-Allo-Isohumulone, Abeo-Isohumulone (oxidierte Isohumulone), Anti-Isohumulone, Spiro-Isohumulone, Humelinsäuren und verschiedene Homologe, wie sie z.B. von M. Verzele: The Chemisty of Hops in "Brewing Science", Herausgeber J.R.A. Pollock, Academic Press, London -New York, 1981, Seiten 279-332, beschrieben werden.

Überraschenderweise wurde gefunden, dass die Zugabe von Humulon und/oder alpha-Isohumulon bzw. von Derivaten derselben, gegebenenfalls in Kombination mit Hopfenölen, zu einer Reihe von festen und flüssigen Lebensmitteln geschmacksverändernd oder geschmacksintensivierend wirkt. Dabei kann das entsprechende Lebensmittel geschmacklich durch das Eigenaroma des Zusatzstoffes verändert werden, oder es kann das Eigenaroma des Lebensmittels intensiviert werden. In manchen Fällen ist es auch möglich, durch die Zugabe der vorstehend genannten Substanzen eine totale Veränderung der Geschmacksrichtung zu bewirken. Darüber hinaus wirkt der Zusatz von Humulon und/oder Isohumulon bzw. Derivaten derselben zu Lebensmitteln und Getränken bakteriostatisch.

Die Zugabe von Humulon und/oder alpha-Isohumulon sowie deren Derivate ist besonders für Getränke geeignet. Diese Getränke umfassen sowohl alkoholfreie als auch alkoholische Getränke, ausgenommen Bier und Ale.

Unter den alkoholfreien Getränken sind insbesondere zu nennen: Fruchtsäfte, vor allem aus Trauben und Zitrusfrüchten sowie Kernobst, Fruchtaromakonzentrate; Fruchtnektare; Fruchtmuttersäfte und Frucht-bzw. Obstsirupe sowie Gemüsesäfte. Besonders geignet ist die Zugabe von Humulon oder Isohumulonen gegebenenfalls in Kombination mit Hopfenölen, zu Orangen-, Grapefruit-und Zitronensäften sowie Kirschsaft. Dabei wird das Eigenaroma des jeweiligen Fruchtsaftes deutlich intensiviert. Es wird ein fruchtartiger Geschmack erzielt, wobei in einigen Fällen die Säure unterdrückt, in anderen die Süsse eines Produktes gedämpft wird.

In gleicher Weise eignet sich die Zugabe von Humulon bzw. Isohumulon, gegebenenfalls in Kombination mit Hopfenölen, zu nicht-alkoholischen Getränken vom Typ der Fruchgetränke, Limonaden und Brausen sowie Essenzen.

Durch die Eigenschaft des Humulons bzw. Isohumulons, die Säure eines fruchtartigen Getränkes zu unterdrücken und/oder demselben teilweise den süssen Geschmack zu nehmen, eignen sich dieselben auch als Zusatz zu weinähnlichen Getränken.

Auch Fruchtsaftpulvern kann man zur Geschmacksintensivierung Humulon bzw. Isohumulon zugeben. Fruchtsaftpulver sind Erzeugnisse, die durch Entfernung nahezu des gesamten in den Fruchtsäften enthaltenen Wassers mit Hilfe physikalischer Verfahren gewonnen werden. Da beim Trockenprozess auch das Aroma weitgehend verlorengeht, sind rückverdünnte Pulver nur dann als vollgültige Fruchtsäfte anzusehen, wenn das Aroma wieder zugeführt wird. Dabei eignen sich Humulon und/oder Isohumulon aufgrund ihrer geschmacksintensivierenden wie auch geschmacksverändernden Wirkung in besonderem Masse, gegebenenfalls im Gemisch mit anderen Aromastoffen.

Im weiteren eignet sich die Zugabe von Humulon und/oder Isohumulon auch bei alkoholischen Getränken, denen dadurch in vielen Fällen ein intensiviertes, bitterndes Aroma verliehen wird. Dabei sind die Spirituosen zu nennen, wie alkoholische Pflanzenauszüge, z.B. Magenbitter und Branntweine, Liköre, Punsche und Mixgetränke.

Darüber hinaus kommen Humulon und Isohumulon auch bei festen Lebensmitteln zur Anwendung. So eignen sie sich z.B., aufgrund ihrer Wirkung als aromatisch und bakteriostatisch wirkende Zusatzstoffe für die Zugabe bei der Brotherstellung.

Vorteilhaft ist auch die Zugabe von Humulon und Isohumulon zu Sossen, insbesondere zu Fleisch-und Salatsossen, da hierbei eine deutliche Verstärkung des Eigengeschmackes hervorgerufen wird. Ausserdem wirkt der Zusatz von Humulon bzw. Isohumulon zu Salatdressings, Ketchup-Sossen, Mayonnaisen, Suppen, insbesondere Trockensuppen, sowie Senf, geschmacksintensivierend.

Die Zugabe von Humulon bzw. Isohumulon eignet sich auch zur Geschmacksintensivierung bei Milchprodukten, wie z.B. Yoghurts, Quark und Fruchtmilch. Bei Yoghurts mit Fruchtzusätzen wird dabei eine starke Intensivierung des Fruchtgeschmackes erzielt. Bei Yoghurts ohne Fruchtzusatz sowie bei Quark, insbesondere Produkten der Magerstufe, wird durch den Zusatz von Humulon ein mehr abgerundeter, rahmähnlicher Geschmack erzielt.

Die Zugabe von Humulon bzw. alpha-Isohumülon, gegebenenfalls in Kombination mit Hopfenölen, erfolgt entweder als ethanolische Lösung oder im Gemisch mit Lösevermittlern. Besonders bevorzugte Lösevermittler sind Glucose, Isoglucose, Fructose, Kornsirupsolids, Dextrine und Gemische derselben. Es ist dabei vorteilhaft, die Zugabe im kalten Milieu durchzuführen, da auf diese Weise die entsprechenden Aromastoffe nicht wieder verloren gehen.

Prinzipiell ist aber auch eine Zugabe von Humulon bzw. Isohumulon und gegebenenfalls Hopfenölen zu einem heissen Medium durchführbar, wie z.B. beim Hoch-Kurzerhitzen und der Heissbfüllung von Fruchtsäften.

Die Konzentration an zugegebenen Humulonen bzw. alpha-Isohumulonsäure und Derivate derselben richtet sich nach dem in einem bestimmten Produkt gewünschten Aroma. Übliche Konzentrationen an Humulonen sind 0,5 bis 150, bevorzugt 1 bis 50 und besonders bevorzugt von 1 bis 20 ppm bzw. mg/l. Übliche Konzentrationen an alpha-Isohumulon und Hopfenölen sind jeweils 3 bis 20, bevorzugt 2 bis 10 mg/l. Bei manchen Lebensmitteln und Getränken kann eine Zugabe von alpha-Isohumulon und Hopfenölen in einer Konzentration bis zu jeweils 100 mg/l erfolgen. Sofern dem Lebensmittel oder Getränk Humulon und/oder Isohumulon und/oder Hopfenöle zugegeben werden, liegt der Konzentrationsbereich dieser Aromastoffe zwischen 0,5 bis 150 ppm, bevorzugt zwischen 1 bis 50 ppm.

Die folgenden Beispiele sollen die gemass der Erfindung mit Hilfe von Humulon-und Isohumulonzusatz erzielten Geschmacksintensivierungen und -veränderungen näher erläutern.

Bei Versuchen wurden jeweils zwei oder drei Proben ausgewertet. Bei der Mehrzahl der Probanden handelte es sich um Normalpersonen, die dem Endverbraucher handelsüblicher Getränke und Lebensmittel entsprechen. Es kann davon ausgegangen werden, dass bei einem trainierten Verkostergremium die Ergebnisse in Richtung Geschmacksintensivierung und Geschmacksveränderung noch deutlicher ausgefallen wären.

Die Zugabe von Humulon erfolgt in Form einer 10%igen ethanolischen Lösung. Jeder Test setzte sich aus 10 Teilnehmern zusammen.

1. Yoghurt ohne Fruchtzusatz:

Es wurden 10 ppm Humulon zugegeben. Die Mehrzahl der Verkoster stellte einen mehr abgerundeten und rahmigen Geschmack des Produktes fest.

2. Heidelbeeryoghurt:

Es wurden 10 ppm Humulon zugegeben. Die Mehrzahl der Verkoster empfand, dass die Süsse des Yoghurts etwas unterdrückt und der eigentliche Yoghurtgeschmack stärker war.

3. Erdbeeryoghurt:

Es wurden 10 ppm Humulon zugegeben. Der Yoghurt wurde als fruchtiger, weniger süss und mehr nach Yoghurt schmeckend empfunden.

Salat-Kräuterdressing:

Es wurden 10 ppm Humulon zugegeben. Die Merhzahl der Verkoster stellte einen deutlichen Unterschied fest, wobei das Dressing mit Humulonzusatz meist als milder, d.h. weniger scharf und sauer, empfunden wurde.

5. Curry-Ketchup:

Es wurden 20 ppm Humulon zugegeben. Sämtliche Verkoster stellten einen deutlichen Unterschied zwischen den Proben fest. Ketchup mit Humulonzusatz wurde als deutlich schärfer und intensiver nach Curry schmeckend beurteilt.

6. Tomaten-Ketchup:

Es wurden 40 ppm Humulon zugegeben. Die Mehrzahl der Verkoster beurteilte den Ketschup mit Humulonzusatz als schärfer mit intensiverem Tomatengeschmack.

7. Süsser Senf:

Es wurden 20 ppm Humulon zugegeben. Auch hier wurde von den meisten Verkostern ein - schärferer und würzigerer Geschmack wahrgenommen.

8. Gulaschsuppe:

Zusatz 60 ppm Humulon. Bei Humulonzugabe wurde ein deutlich schärferer bzw. bitterer Geschmack wahrgenommen.

9. Ochsenschwanzsuppe:

Zugabe von 10 ppm Humulon. Von der Mehrzahl der Verkoster wurde die Suppe aufgrund ihres intensiveren Aromas als besser beurteilt.

10. Mayonnaise:

Zusatz 150 ppm Humulon. Die Mehrzahl der Verkoster empfand das Produkt mit Zusatz - schärfer, pikanter und aromatischer.

Die weiteren Versuche betreffen die Zugabe von Isohumulon, wobei dasselbe als 10%ige ethanolische Lösung zugegeben wurde. Auch hier setzte sich jeder Dreieckstest aus 10 Teilnehmern zusammen.

11. Orangensaft (handelsübliches Produkt):

Zugabe: 3 ppm Isohumulon

Wirkung: deutliche Verstärkung des Fruchteigenaromas, Abschwächung der Süsse und Säure;

Dreiglastest: 10 Teilnehmer

7 x richtig

3 x falsch

12. Schweppes Bitter Orange:

Zugabe: 2 ppm Isohumulon

Wirkung: Aroma in Richtung Grapefruit verändert;

Dreieckstest: 10 Teilnehmer

7 x richtig

3 x falsch

13. Kirschsaft (handelsübliches Produkt, 100%ig):

Zugabe: 3 ppm Isohumulon

Wirkung: verstärkter aromatischer Geruch und intensiviertes Fruchtaroma

Dreieckstest: 10 Teilnehmer

8 x richtig

2 x falsch

14. Traubensaft (handelsübliches Produkt):

Zugabe: 8 ppm Isohumulon

Wirkung: Veränderung von Süsse, Säure und Fruchtaroma, bitterer Nachgeschmack, (es war eine hohe Zugabe nötig, um einen deutlichen Unterschied zu erkennen);

Dreieckstest: 9 Teilnehmer

4 x richtig

5 x falsch.

## Ansprüche

1. Aromazusatz zu Lebensmitteln und Getränken, ausgenommen Bier, dadurch **gekennzeichnet** , dass dieser Humulon und/oder alpha-Isohumulon bzw. ein Derivat derselben umfasst.

2. Bakteriostatischer Zusatz zu Lebensmitteln und Getränken, ausgenommen Bier, dadurch **gekennzeichnet** , dass dieser Humulon und/oder alpha-Isohumulon bzw. ein Derivat derselben umfasst.

3. Zusatz gemäss den Ansprüchen 1 und 2, dadurch **gekennzeichnet** , dass dieser im weiteren Hopfenöle umfasst.

4. Zusatz gemäss den Ansprüchen 1 und 2, dadurch **gekennzeichnet** , dass dieser im weiteren einen Lösevermittler umfasst.

5. Zusatz gemäss Anspruch 4, dadurch **gekennzeichnet** , dass der Lösevermittler Glucose, Isoglucose, Fructose, Kornsirupsolids, Dextrine oder Gemische derselben darstellt.

6. Zusatz gemäss den Ansprüchen 1 und 2, **gekennzeichnet** durch eine ethanolische Lösung von Humulon und/oder alpha-Isohumulon bzw. Derivaten derselben.

7. Verwendung des Aromazusatzes gemäss den Ansprüchen 1 bis 6 zur Intensivierung des Eigengeschmackes, zur Geschmacksveränderung, zur Unterdrückung des sauren Geschmackes und zum Bittern von Lebensmitteln und Getränken, ausgenommen Bier.

8. Verwendung des Aromazusatzes gemäss den Ansprüchen 1 bis 6 in nicht-alkoholischen Getränken, insbesondere Fruchtsäften, und alkoholischen Getränken, ausgenommen Bier und Ale.

9. Verwendung des Aromazusatzes gemäss den Ansprüchen 1 bis 6 in Sossen, Mayonnaisen, Salatdressings, Ketchup, Senf sowie in Milchprodukten, insbesondere Yoghurts.

10. Verwendung des Aromazusatzes gemäss den Ansprüchen 1 bis 6 bei der Brotherstellung.